# EUROPÄISCHE PATENTANMELDUNG

(11) **EP 0 909 563 A1**
(43) Veröffentlichungstag der Anmeldung: **21.04.1999**
(21) Anmeldenummer: 97118023.7
(22) Anmeldetag: 17.10.1997
(51) Int. Cl.: A61K 35/78

(54) **Therapie von chronischen entzündlichen Darmerkrankungen mit einer Phytokombination**

(71) Anmelder: OMER, Osama L. M., Dr.Dr., 79618 Rheinfelden (DE)
(72) Erfinder: OMER, Osama L. M., Dr.Dr., 79618 Rheinfelden (DE)

(57) **Zusammenfassung**

Die Erfindung betrifft Heilpflanzen für die Behandlung von unspezifischen Entzündungen des Darmes. Dazu gehören u.a. Morbus Crohn und die Colitis Ulcerosa. Wermutkraut (Artemesia absinthium) und/oder Cardamomfrucht fructus elettaria cardamomum) und/oder Rosenblüten flores rosae) und/oder Mastix-Resin (resin pistacia lenticus) in Trockenpulver- oder Extraktform besitzen die gewünschte therapeutische Wirkung.

## Beschreibung

Zu den chronischen entzündlichen Immunerkrankungen des Darms gehören die Colitis ulcerosa, der Morbus Crohn, der auch als Colitis granulomatosa bezeichnet wird, und in weiterem Sinne auch Colon Irretables und Divertikulitis. Sie werden auch unter den Begriff der "unspezifischen entzündlichen Darmerkrankungen" zusammmengefaßt. Die Ursachen dieser Erkrankungen setzten sich aus zum Teil bekannten, zum Teil noch unklaren Faktoren zusammen. Coltis granulomatosa (M. Crohn) und Colitis ulcerosa sind klinisch miteinander verwandt und treten manchmal (ca 10%) gemeinsam auf.

Im Darmbereich laufen zahlreiche Immunreaktionen ab, die in erster Linie als Schutzfunktion des Immunsystems zu verstehen sind. Klinische und immunologische Befunde lassen vermuten, daß die Fehlsteuerung der Immunreaktionen ursächlich oder reaktiv bei der Entstehung und dem Ablauf dieser chronischen entzündlichen Darm-Erkrankungen beteiligt sind. Es finden sich im peripheren Blut, in den lymphatischen Geweben und in der Darmwand entscheidende Hinweise für Zeichen der humoralen wie der zellvermittelten Immunabwehr gegen Antigene des Colon, häufig auch gegen andere körpereigene Substanzen wie Kernbestandteile, Blutzellen und Zellen der Schleimhäute sowie gegen Antigene die von außen in den Intestinaltrakt hineingelangen. Während bei der Crohn'schen Erkrankung eher die überschießende T-Zellenaktivität im Vordergrund steht, sind es bei der Colitis ulcerosa eher die überschießenden Antikörperreaktionen.

Für die Behandlung dieser Erkrankungen stehen Kortikoide, Salicylazosulfapyridine (Azulfidine) und Immunsupressiva wie Azathioprin zur Verfügung. Da diese Erkrankungen in Schüben verlaufen und zu Rezidiven neigen, die oft nicht voraussehbar sind, sollte es das Ziel der Therapie sein, den einzelnen Schub möglichst schnell (innerhalb einer Woche) und möglichst frei von Nebenwirkungen zu behandeln. Ziel müßte auch eine Langzeit-Remission und Besserung des allgemeinen Befindens sein. Kortikoide wirken ohne Zweifel in der akuten Phase rasch, eignen sich aber für die Langzeittherapie kaum. Die Nebenwirkungen einer Langzeit-Kortikoidtherapie sind voller Gefahren und oft nicht vertretbar. Die Azulfidine wirken deutlich langsamer als die Kortikoidtherapie. Zu den Azulfidine ist zu sagen, daß echte Heilungserfolge dadurch nicht zu erreichen sind und daß der Enthusiasmus der vergangenen Jahre mit diesem Therapeutikum zurückgegangen ist. Immunsupressive Medikamente kommen lediglich bei Therapieversagen mit Azulfidine oder Kortikoiden in Betracht. Sie sind wegen schwerwiegender Nebenwirkungen - Risiko einer Knochenmark-supression, gehäufte Neoplasien - für die allgemeine Anwendung nicht zu empfehlen.

Wirksame Phytopharmaka sind wegen fehlender Nebenwirkungen synthetisch hergestellten Arzneien vorzuziehen. Für die Behandlung von chronischen entzündlichen Darmerkrankungen aber steht außer Opium keine Phytotherapie zur Verfügung. Die Erfindung hat sich die Aufgabe gestellt, eine risikofreie, rasch einsetzende und langanhaltende phytotherapeutische Alternative für chronische entzündliche Darmerkrankungen zu schaffen. Diese Aufgabe ist mit folgenden Pflanzen und ihren Kombinationen gelungen:
Wermutkraut (artemesia absinthium vulgaris)
Mastix-Resin (resin pistacia lenticus)
Cardamomfrucht (fructus elettaria cardamomum)
Die o.g. Pflanzen bzw. ihre Teile sind bekannte Drogen. Nicht bekannt ist ihre Anwendung bei unspezfischen Entzündungen des Darmes wie Morbus Crohn, Colitis Ulcerosa, Divertikulitis und Reizdarm. Die Erfindung beschreibt die antientzündliche Wirkung von Wermutkraut, Mastixresin, Kardamomfrucht und Rosenblüten einzeln und in Kombination untereinander.

Um die gewünschte therapeutische antientzündliche Wirkung zu demonstrieren wurden nachfolgende Pflanzen bzw. deren Mischungen getestet:
Gruppe 1. Wermutkraut
Gruppe 2. Mastixresin
Gruppe 3. Cardamomfrucht
Gruppe 4. Wermutkraut und Mastixresin
Gruppe 5. Wermutkraut und Cardamomfrucht
Gruppe 6. Wermutkraut, Mastixresin und Cardamomfrucht
Gruppe 7. Gruppe 5 + Rosenblüten
Gruppe 8. Mastixresin und Cardamomfrucht
Gruppe 9. Rosenblüten und Cadamomfrucht und Mastix-Resin
Gruppe 10 Extraktmischung von Mastix-Resin und Wermutkraut und feingemahlene Rosenblüten.

Diese Pflanzen bzw. Pflanzenteile und deren Mischungen wurden getrocknet, mit UV-Licht desinfiziert, feingemahlen, versiebt (>1mm Größe) und in Verbindung mit üblichen Applikationsträgern als Hartgelatinekapseln vorbereitet. Ethanol diente als Extraktionsmittel für Wermutkraut und Mastix-Resin.

Um einen Anhaltspunkt für die Anti-Morbus Crohn-Aktivität zu bekommen, wurden Patienten die an einem Rezidiv dieser Erkrankung litten, mit den Pflanzen bzw. dem Pflanzengemisch behandelt. Die Diagnose wurde anhand des klinischen Erscheinungsbildes, dem typischen Röntgenbild und des histologischen Befundes gestellt. Vor der Aufnahme in die Studie wurde das Einverständnis des Patienten eingeholt. Eine Anti-Crohn-Aktivität wurde angenommen, wenn bei einem frischen, nicht länger als 2-3 Tage altem Rezidiv die typischen Schmerzen und Durchfälle innerhalb von 3-4 Tagen eine deutliche Rückbildungstendenz zeigten.

Es wurde herausgefunden, daß die einzelnen Pflanzen - Wermutkraut, Mastix-Resin, Rosenblüten und Cardamomfrucht eine schwache Anti-Crohn-Aktivität besitzen. Sie entfalten aber in Kombination miteinander, insbesondere wenn Wermutkraut ein Bestandteil dieser Kombination ist, eine deutliche, klinisch nutzbare Wirkung. Als besonders ausgewogen und optimiert zur Behandlung von Crohn-Erkrankungen zeigten sich die Mischungen der Gruppen 6 und 7. Schwach in der Wirkung waren die Gruppen 8, 9 und 10 sowie die Gruppen 1 bis 3.

Die Erfindung wird anhand der folgenden Beispiele erläutert:

Die einzelnen, unabhängig voneinander getrockneten, zu feinem Pulver gemahlenen und gesiebten Ausgangsmaterialien wurden miteinander gemischt, um das u.g. Erfindungsgemisch A zu ergeben. Das Feinpulver wurde in Kapseln der Größe 0 abgefüllt, in denen das Gewicht der Mischung von 325 mg pro Kapsel erhalten wurde.

Erfindungsgemisch A
- Wermutkraut: 200 mg
- Rosenblüten: 100 mg
- Cardamomfrucht: 25 mg
- 50 mg Mastix-Resin: diente als Trägersubstanz
- Gesamtgewicht der Kapsel:: 375 mg (Tagesdosis 3x3 Kapseln)

### Beispiel 1

### Anwendung bei Colitis granulomatosa (Morbus Crohn)

### Selektion des Patientenguts

Patienten mit der gesicherten Diagnose von Morbus Crohn wurden mit dem erfindungsgemäßen Gemisch A behandelt. Die Diagnose von Morbus Crohn wurde anhand von folgenden Kriterien gestellt:
a) Klinisches Bild und Anamnese: (anhaltende, uncharakteristische Bauchschmerzen mit Durchfällen, Fieber, Gewichtsverlust, perianale Fisteln, Fissuren und unklarer Gelenk- und Augensymptomatik wie Iritis, Episkleritis).
b) Röntgenbild (typische segmentale Stenosen mit Wandstarre, Pflastersteinrelief, asymmetrischer Befall mit Raffung des Mesentriums und sackartigen Ausstülpungen der gegenüberliegenden Wand)
c) Rekto-Koloskopie (umschriebene Hyperämie, aphtoide Läsionen, längs- und quergestellte Ulzerationen, Fissuren und/oder perianale Fisteln)
d) Histologie (in histolgischen Biopsie-Präparaten eine diffuse, entzündliche Infiltration der Darmwand, die aus Lymphozyten, Plasmazellen und eosinophilen Granulozyten bestehen, die typischerweise die Muskolaris mucosae durchbricht und auf die Submukosa sowie die Muskolaris propria übergreift. Eine follikuläre, lymphatische Hyperplasie bis hin zum subserösen Fettgewebe, eine oberflächliche Ulzeration der Schleimhäute. Profilative, granulierende Entzündung und Boeck-ähnliche Granulome mit Epitheloidzellen und Langhan'schen Riesenzellen.)

### Methodik des Versuchs

Der Versuch wurde angelegt als randomisierte, prospektive doppelblinde Studie mit parallelen Gruppen, nach den Richtlinien der Deklaration von Helsinki und Good Clinical Practice (GCP). Alle Patienten gaben nach Aufklärung ihre Einwilligung. Als Vergleichssubstanz wurde Sulfasalzin (Azulfidine) verwendet. Um die Doppelblindheit des Versuchs zu sichern, wurden sowohl die erfindungsgemäße Pflanzenmischung A als auch die Sulfasalzinetabletten (500mg) gemahlen und in dunklen Hartgelatinekapseln abgefüllt. Die Kapseln hatten völlig identisches Aussehen. Die Patienten wurden doppelblind randomisiert den einzelnen Behandlungen zugeordnet. Die teststatistische Auswertung bei einer zweiseitigen Alternativhypothese mit einer Irrtumswahrscheinlichkeit von a= 0,05 erfolgte für folgende Parameter
- Schmerz am Tag 2, 4, 6, 10 und 14
- Zahl der Durchfälle am Tag 2, 4, 6, 10 und 14
Die Studie sollte für jeden Patient 14 Tage dauern. Es wurden 3x3 Kapsel pro Tag verabreicht. Die Wirksamkeit der Behandlung wurde angenommen, wenn innerhalb einer Woche die klinischen Symptome wie Magenschmerzen, Fieber und Durchfälle eine deutliche Remission zeigten.

### Ergebnisse des Versuchs

Vierzig Patienten mit gesicherter Morbus-Crohn-Erkrankung wurden zur Zeit eines akuten Rezidivs in den Versuch aufgenommen und entweder mit dem erfindungsgemäßen Gemisch A (Gruppe A) oder mit Azulfidine behandelt (Gruppe B). In jeder Gruppe waren 20 Patienten. Die Geschlechts- und Altersverteilung war in den beiden Gruppen unterschiedlich, statistisch jedoch nicht signifikant. Alle Patienten der Gruppe A waren zwischen dem 4. und 7. Tag weitgehend beschwerdefrei, in der Azulfidine Gruppe jedoch war dies erst zwischen den 7. und 10. Tag der Fall. Nach etwa 10 Tagen hatte keiner der Patienten der Gruppe A mehr Durchfälle und nach 14 Tagen auch keine Schmerzen mehr. In der Gruppe B war die Schmerzfreiheit nur bei etwa der Hälfte der Patienten erreicht.

Die Ergebnisse belegen eindeutig die Wirksamkeit des erfindungsgemäßen Gemischs A bei der Behandlung von Morbus Crohn. Die Erfindung ist nicht nur wirksam, sondern der Standardtherapie mit Azulfidine sogar überlegen.

### Beispiel 2

### Anwendung bei Colitis Ulcerösa

### Selektion des Patientenguts

Die Colitis Ulcerosa gehört wie der Morbus Crohn zu den chronischen unspezifischen Darmenzündungen unbekannter Ursache. Sie betrifft primär Mukosa und erfaßt selten tiefere Wandschichten. Typisch ist die von distal nach proximal ausgedehnte Lokalisation im Kolon. Das Rektum ist stets befallen. Leitsymptome sind blutige, eitrige Stühle. Zehn Patienten mit der gesicherten Erkrankung von Colitis Ulcerosa wurden mit dem erfindungsgemäßen Gemisch A behandelt. Die Diagnose von Colitis Ulcerosa wurde anhand von folgenden Kriterien gestellt:
a) Klinisches Bild (anhaltende, uncharakteristische Bauchschmerzen mit blutigen, eitrigen Stühle, die sich langsam steigern und schließlich in reine Blutstühle übergehen. Fieber, Gewichtsverlust).
b) Röntgenbild (fein granuliertes Schleimhautrelief (Schummerung), verminderte Wandelastizität, dornförmige Wandvertiefung - Spiculae, ödematös geschwollene Schleimhautinseln. Starres, enggestelltes Darmrohr)
c) Rekto-Koloskopie (typisches Schleimhautödem, Oberfläche granulär statt glatt, Pseudopolypen, Hyperämie, vermehrte Lädierbarkeit, diffuse Blutungen, multiple Ulzeration ohne scharfe Abgrenzung einzelner Ulzera)
d) Histologie (in histologischen Biopsie-Präparaten eine ausgeprägte entzündliche Infiltration von Stroma und submukosa, Gefäße der Submukosa sind nicht sichtbar)

### Methodik des Versuchs

Der Versuch wurde angelegt als offene Studie nach den Richtlinien der Deklaration von Helsinki und Good Clinical Practice (GCP). Alle Patienten gaben nach der Aufklärung ihre Einwilligung. Die Behandlung mit dem erfindungsgemäßen Gemisch wurde 14 Tage lang fortgesetzt. Sofern am 14. Tag keine Besserung der Stuhlqualität erreicht war, wurden die Patienten aus der Studie entlassen.

### Ergebnisse des Versuchs

Zehn Patienten wurden in die Studie aufgenommen. Alle zeigten ab dem 10. Tag eine deutliche Besserung ihrer klinischen Symptomatik, die der Kortikoid-Behandlung der Vergangenheit vergleichbar war.

## Patentansprüche

1. Verwendung von Wermutkraut (Artemesia absinthium) und/oder Cardamomfrucht fructus elettaria cardamomum) und/oder Rosenblüten flores rosae) und/oder Mastix-Resin (resin pistacia lenticus) in Trockenpulver- oder Extraktform und in Verbindung mit den üblichen Applikationsträgern zur Herstellung eines Arzneimittels zur Behandlung von unspezifischen Entzündungen des Darmes.
